# FASCICULE DE BREVET EUROPEEN

(11) **EP 1 833 550 B1**
(45) Date de publication et mention de la délivrance du brevet: **12.04.2017**
(21) Numéro de dépôt: 05819309.5
(22) Date de dépôt: 02.12.2005
(51) Int. Cl.: A61B 17/34, A61M 39/28, A61M 39/06

(54) **VALVE POUR INSTRUMENT CHIRURGICAL OU MEDICAL**
VENTIL FÜR EIN CHIRURGISCHES ODER MEDIZINISCHES INSTRUMENT
VALVE FOR A SURGICAL OR MEDICAL INSTRUMENT

(30) Priorité: 06.12.2004 FR 0452874; 08.04.2005 US 669361 P
(43) Date de publication de la demande: 19.09.2007
(73) Titulaire: Mialhe, Claude, 83300 Draguignan (FR)
(72) Inventeur: Mialhe, Claude, 83300 Draguignan (FR)
(74) Mandataire: Decobert, Jean-Pascal
(86) Numéro de dépôt international: PCT/EP2005/056440
(87) Numéro de publication internationale: WO 2006/061356

(56) Documents cités:
- WO-A-03/048616
- US-A- 5 211 370
- US-B1- 6 221 057
- US-B1- 6 808 520

## Description

La présente invention concerne une valve pour instrument chirurgical ou médical ainsi qu'un instrument chirurgical ou médical équipé de ladite valve.

L'invention trouvera en particulier son application pour la réalisation d'introducteur endovasculaire.

Ce domaine n'est cependant pas limitatif.

Les instruments d'introduction dans le corps et en particulier pour les interventions endovasculaires y compris percutanés et/ou transpariétals nécessitent la présence d'éléments d'obturation aptes à assurer une étanchéité de l'introducteur.

Plus particulièrement, il s'agit lors de l'introduction d'objets tels des objets allongés comme des guides chirurgicaux, ballons et endoprothèses dans l'instrument d'éviter autant que faire se peut la fuite rétrograde de flux sanguins au travers de l'instrument introducteur.

Le document US-A-5.256.150 présente un dispositif d'introduction de cathéters dans un vaisseau du corps humain.

Ce dispositif s'applique à un système de très large diamètre avec un élément cylindrique déformable en matériau flexible apte à être déformé en torsion par rotation relative de ces deux extrémités.

Cette déformation est induite par rotation d'une des extrémités au moyen d'un système d'entraînement complexe comprenant une crémaillère et un système de roues dentées.

Un tel dispositif ne donne pas entière satisfaction notamment parce que le système de transmission de mouvement entre la main de l'opérateur et l'élément cylindrique déformable en torsion est complexe ce qui induit un coût de fabrication important et génère une construction volumineuse.

Le document US6808520 B1 divulgue une gaine d'introducteur avec une valve souple déformable en torsion, avec un actionnement en torsion comportant une partie de préhension et des moyens de transmission permettant d'entraîner en rotation une partie de la valve.

La publication WO 03048616 A1 montre une autre réalisation de valve avec un élément souple.

US 5211370 divulgue une valve sous forme d'une paroi fine et élastique qui peut être déformée.

La présente invention permet de remédier en tout ou partie aux inconvénients des dispositifs actuels et présente une alternative aux moyens d'actionnement connus.

Un des avantages de l'invention est de permettre la réalisation d'un système de valve nettement moins cher que ceux existants et beaucoup moins volumineux.

L'encombrement de l'ensemble d'actionnement est particulièrement important car plus le système est encombrant, plus l'ergonomie est diminuée.

Suivant un mode de réalisation, de par sa configuration, la valve selon l'invention peut être actionnée par un opérateur au moyen d'une seule main, la main qu'il utilise pour tenir l'instrument chirurgical lui-même.

L'invention permet également, suivant une variante préférée, de réaliser un déplacement en rotation d'un élément déformable en torsion de façon continue sans aucun à-coup.

Par ailleurs, l'entraînement par câble proposé suivant le mode de réalisation préféré de l'invention permet de positionner et d'orienter les moyens de préhension par l'utilisateur de façon très souple et très flexible suivant ce qui est souhaité en terme de facilité d'utilisation.

On peut par ailleurs parfaitement s'adapter à la taille de l'élément à introduire pour s'appliquer sur cet élément avec une excellente étanchéité.

En particulier, le système selon l'invention autorise un actionnement par l'utilisateur orienté dans le sens longitudinal de la valve.

Cela permet de produire un actionnement naturel.

L'invention concerne une valve pour instrument chirurgical ou médical comportant :
- un passage au moins partiellement obturable par déformation en torsion d'une portion souple de la paroi du passage,
- des moyens d'actionnement en rotation d'une extrémité mobile de la portion souple aptes à engendrer la torsion, caractérisée par le fait que les moyens d'actionnement comportent un câble de transmission de mouvement à l'extrémité mobile de la portion souple.

Suivant des variantes non limitatives mais avantageuses, cette valve est telle que :
- un bout du câble de transmission est fixé à un moyen de préhension déplaçable par un opérateur,
- il comporte un organe de renvoi d'angle pour dévier le câble de transmission,
- l'organe de renvoi d'angle est une poulie,
- l'organe de renvoi d'angle et le moyen de préhension sont configurés pour que le moyen de préhension soit déplaçable en translation selon l'axe longitudinal de la valve,
- elle comporte des moyens de rappel en position par défaut de l'extrémité mobile,
- les moyens de rappel sont un ressort de torsion dont une extrémité est fixe et l'autre extrémité est solidaire en rotation de l'extrémité mobile de la portion souple,
- les moyens de rappel sont configurés pour obturer le passage de la valve par défaut,
- elle comporte des moyens de blocage de la valve en position ouverte,
- les moyens de blocage comportent une goupille d'arrêt des moyens d'actionnement,
- l'extrémité mobile est solidaire en rotation d'un arbre tournant auquel est fixé un bout de câble de transmission,
- l'arbre tournant comporte une gorge de fixation et d'enroulement du câble de transmission,
- elle comporte un câble complémentaire apte à transmettre, lorsqu'il est sollicité en traction, un mouvement à l'extrémité mobile de la portion souple dans un sens inverse à celui du câble de transmission,
- le câble complémentaire est guidé par une poulie de retour et une poulie de renvoi d'angle,
- l'extrémité mobile est solidaire en rotation d'un arbre tournant comportant une gorge de fixation et d'enroulement du câble de transmission et une gorge additionnelle de fixation et d'enroulement du câble complémentaire,
- le moyen de préhension est déplaçable en translation dans une glissière dont les bords comportent au moins une rangée de dents de rétention d'un doigt monté élastiquement sur le moyen de préhension.

La présente invention concerne également un instrument médical ou chirurgical équipé d'une telle valve.

D'autres buts et avantages apparaîtront au cours de la description qui suit d'un mode préféré de réalisation de l'invention qui n'est cependant pas limitatif.

Les dessins ci-joints sont donnés à titre d'exemples et ne sont pas limitatifs de l'invention. Ils représentent seulement un mode de réalisation de l'invention et permettront de la comprendre aisément.

Les figures 1 à 3 illustrent la portion souple de la valve dans trois configurations de sollicitation mécanique en torsion.

La figure 4 est une vue suivant la ligne de coupe B-B de la figure 5 permettant de présenter longitudinalement les éléments composant la valve.

La figure 5 est une vue de côté de l'invention.

Les figures 6 à 9 illustrent tour à tour certains composants de la valve de l'invention.

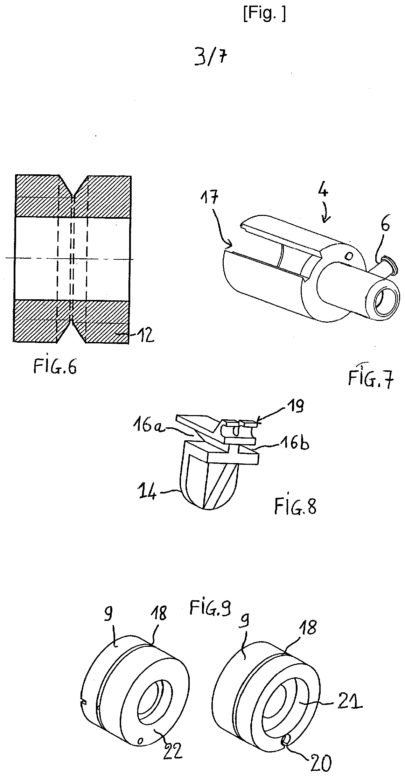

Ainsi, la figure 6 montre une poulie utilisable comme organe de renvoi d'angle.

La figure 7 montre un exemple de configuration du corps de valve.

La figure 8 illustre une possibilité de moyens de préhension.

La figure 9 montre une réalisation de l'arbre tournant en perspective avec une vue montrant plus particulièrement la face avant et une vue montrant plus particulièrement la face arrière de l'arbre.

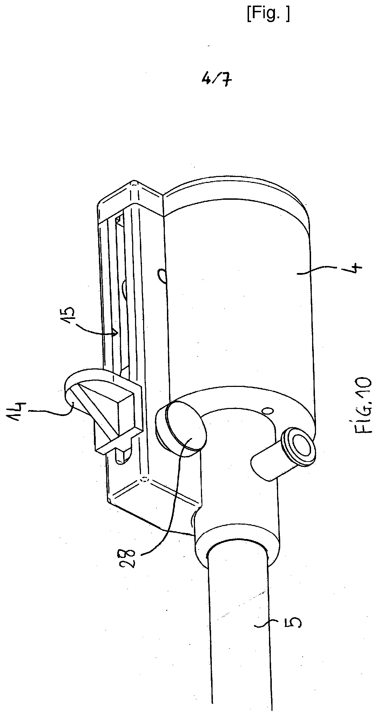

La figure 10 illustre une vue en perspective de l'invention.

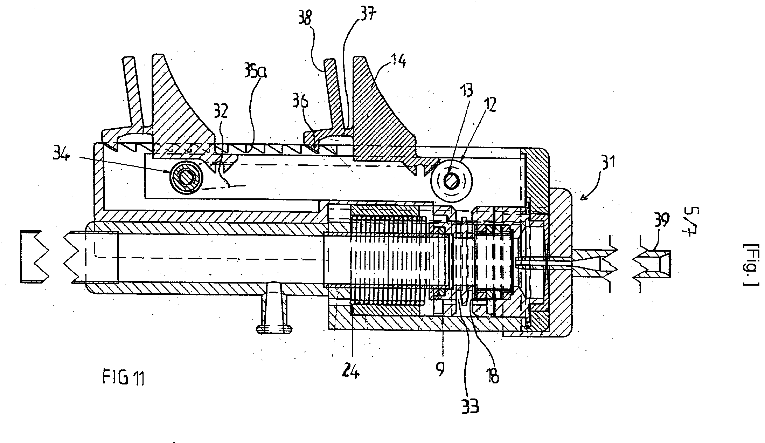

La figure 11 illustre un autre mode de réalisation de l'invention, avec un système de deux câbles antagonistes.

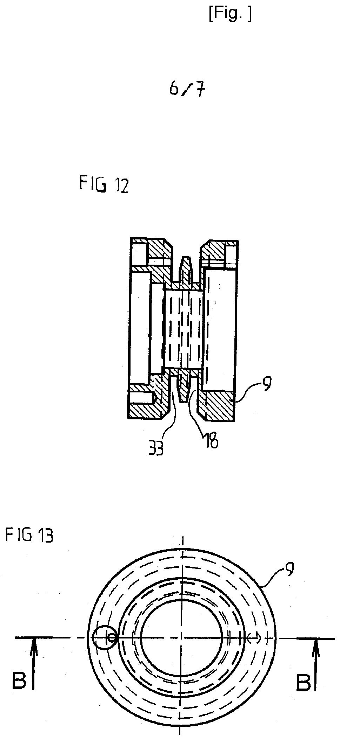

La figure 13 montre une vue de face d'un arbre tournant utilisable dans ce mode de réalisation et la figure 12 en est une vue de coupe.

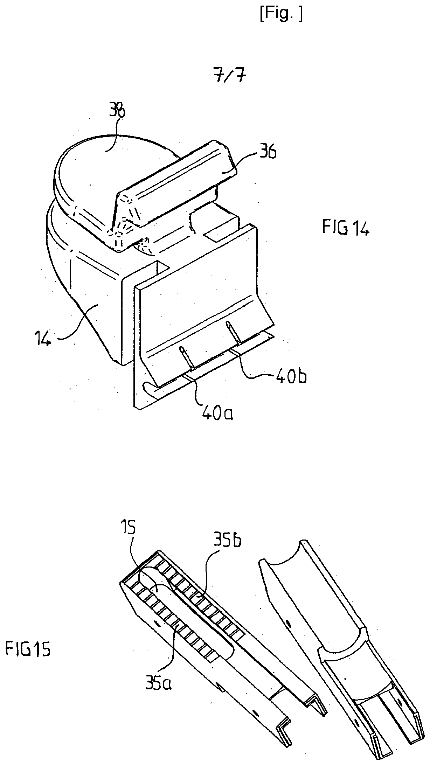

La figure 14 présente une configuration d'un moyen de préhension servant à accrocher une extrémité des câbles.

Une glissière de coulissement du moyen de préhension de la figure 14 est représentée en figure 15.

La valve selon l'invention utilise les propriétés de déformation en torsion d'une portion souple 1 d'un passage interne à la valve.

Ce passage s'étend d'une façon générale suivant la longueur de la valve pour déboucher dans un tube 5 dont une partie distale participe à l'introduction dans le corps.

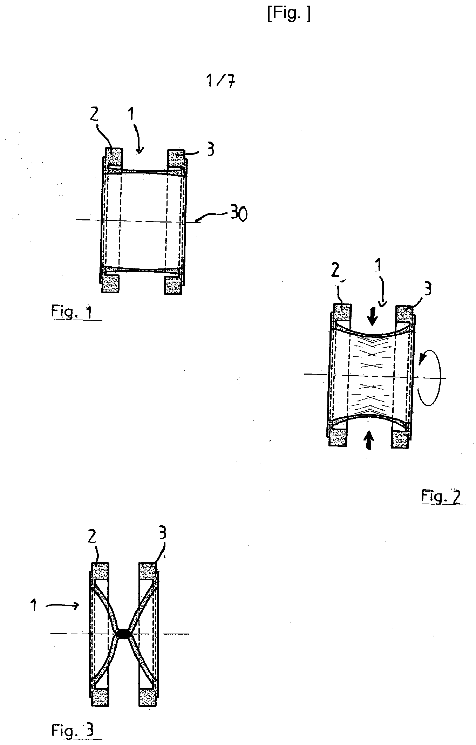

La portion souple telle qu'apparaissant aux figures 1 à 3 est constituée sous forme sensiblement cylindrique creuse en matériau étanche et flexible tel du silicone.

La portion 12 comprend ici des extrémités 2, 3 présentant un rebord plus épais que le reste de la portion souple 1 de sorte à constituer des éléments de solidarisation de la portion souple avec d'autres pièces.

Dans le cas de la figure 1, la portion souple 1 n'est pas sollicitée et le passage situé en son centre est complètement ouvert.

En figure 2, une flèche suivant l'axe de la valve indique la mise en rotation de l'extrémité 3 et, partant, un début de torsion de la portion souple 1 tendant à créer une zone de striction sensiblement au milieu de la longueur de la portion souple 1 comme l'indiquent les flèches orientées verticalement.

La figure 3 montre une autre configuration de la portion souple 1 dans lequel la torsion a été poursuivie de sorte à obturer complètement le passage au centre de la portion souple 1.

On décrit maintenant plus précisément les moyens permettant de mettre en rotation l'extrémité 3 de la portion souple 1 de façon pratique.

On notera que l'exemple ici donné réalise une mise en rotation de l'extrémité 3.

Il pourrait cependant s'agir de l'autre extrémité.

Par ailleurs, une rotation simultanée et opposée des deux extrémités 2, 3 n'est pas exclue par l'invention.

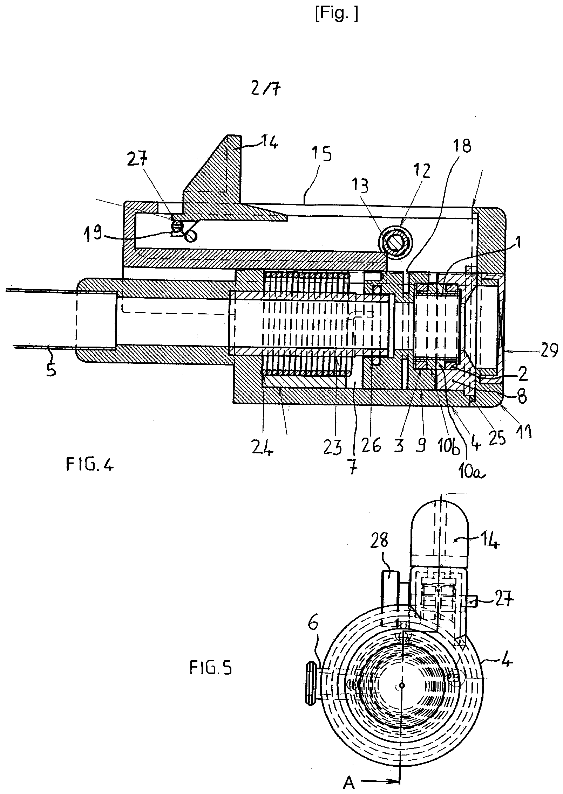

La figure 4 présente un exemple préféré de réalisation des moyens d'actionnement en rotation de l'extrémité 3 de la portion souple 1 employant un câble.

A cette figure, le passage au centre de la valve est fermé ce qui constitue avantageusement la position par défaut du système.

Dans ce cadre, l'extrémité 3 de la portion souple 1 est tournée par défaut de sorte à tordre la portion souple 1.

L'actionnement par l'opérateur permet de tourner l'extrémité 3 dans le sens de l'ouverture lorsqu'il souhaite introduire un objet au travers de la valve.

Dans le cas illustré, l'extrémité 3 est solidaire en rotation d'un arbre tournant 9 présenté en figure 4 et illustré plus en détail en figure 9.

L'extrémité 3 est reçue au niveau de la face avant 21 de l'arbre tournant 9 et sa fixation est assurée par une bague interne 10b appliquée contre le rebord de l'extrémité 3.

Ce montage apparaît en figure 4.

De façon relativement semblable, l'extrémité 2 est solidarisée à une bague fixe 8 recevant sur l'une de ses faces l'extrémité 2 sur laquelle vient se positionner une bague interne 10b de sorte à la bloquer par son rebord.

L'extrémité 2 est dans le cadre représenté, située à l'extrémité proximale de la valve.

L'ensemble est reçu dans un corps 4 par exemple constitué en matière plastique moulé et fermé par un capot 11 préférentiellement doté d'un joint d'étanchéité 25 assurant la fermeture étanche de l'ensemble.

Le joint d'étanchéité 25 comprend une découpe centrale permettant le passage des objets et leur centrage.

Cette étanchéité n'est cependant pas parfaite et elle est optimisée par celle produite par la portion souple 1.

L'arbre tournant 9 entraîne en rotation l'extrémité 3 de la portion souple 1.

Cet arbre 9 est lui-même entraîné par un câble non représenté, déplacé sous l'action de l'utilisateur par le biais de moyens de préhension 14 ici sous forme de tirette déplaçable en translation suivant l'axe longitudinal de la valve.

Les moyens de préhension 14 tels qu'illustrés aux figures 4, 5 et 8 présentent une partie de préhension par l'utilisateur, une zone de fixation 19 permettant la solidarisation d'un bout du câble et une partie intermédiaire dotée de deux rainures 16a, 16b aptes à coopérer avec une glissière 15 formée longitudinalement sur une portion latérale du corps 4.

La portion latérale constituant la glissière peut être constituée d'un seul tenant dans le corps 4 ou réalisée sous forme de pièce rapportée.

La glissière comprend une ouverture dont les bords longitudinaux permettent l'engagement des rainures 16a, 16b et le coulissement des moyens de préhension 14.

Dans le cas où la glissière 15 est rapportée sur l'ensemble du corps 4, le corps 4 présente une découpe 17 permettant le montage de la partie formant glissière telle que visible en figure 7.

Le câble fixé au niveau de la zone de fixation 19 transite par un organe de renvoi d'angle illustré sous forme de poulie 12 tournant autour d'un axe 13 en figure 4.

Le renvoi d'angle assure l'orientation tangentielle du câble pour sa fixation au niveau de l'arbre tournant 9.

Plus précisément, l'arbre 9 comporte une gorge 18 dans laquelle est fixée l'autre bout du câble.

Lors de la rotation de l'arbre 9, le câble est enroulé ou déroulé dans la gorge 18.

La fixation du bout de câble dans l'arbre 9 peut s'effectuer par l'intermédiaire d'un ergot engagé au travers de la gorge 18 par exemple par un trou 20.

On comprend aisément que l'action de l'opérateur sur les moyens de préhension 14 dans le sens longitudinal de la valve assure une traction sur le câble qui, tout en passant sur la poulie 12, exerce un effort apte à mettre en rotation l'arbre tournant 9 et en conséquence, l'extrémité 3 de la portion souple 1.

Comme indiqué précédemment, la valve est avantageusement configurée par défaut pour être obturée.

Dans ce cadre, un moyen de rappel en position obturée par défaut est réalisé.

Dans le cas illustré, il s'agit d'un ressort de tension 24 orienté longitudinalement dans le sens de la valve et reçu autour d'un élément de guide 23 réalisé sous forme d'un cylindre creux préservant un passage central.

L'une des extrémités du ressort de torsion 24 est fixe relativement au corps 4.

L'autre extrémité est solidaire de la rotation de l'arbre tournant 9.

Comme visible en figure 4, le corps 4 comprend une cavité 7 permettant la réception du ressort de torsion 24 et de son guide 23.

Par ailleurs, le pourtour de la cavité 7 forme un organe de guidage en rotation de l'arbre tournant 9.

A titre accessoire, le corps 4 peut être muni d'une ouverture latérale 6, par exemple pour l'apport additionnel de fluide tel un anticoagulant, un produit de contraste ou de lavage.

Dans le cas représenté, et en particulier tel que visible aux figures 4, 5 et 10, des moyens de blocage de la valve en position ouverte sont présents. En effet, lors d'une longue période de non utilisation (notamment au stockage), il est avantageux que la portion souple 1 ne soit pas sollicitée en torsion pour éviter la dégradation de ses propriétés élastiques (notamment collage si portion souple en silicone).

Dans le cas représenté, les moyens de blocage comportent une goupille 27 apte à former une butée de rétention du moyen de préhension 14. Dans la position de la figure 4, la portion souple 1 est ainsi ouverte alors que le ressort de torsion 24 est en travail.

On notera que les moyens de blocage de l'invention sont utilisables avec divers types de moyens d'actionnement en rotation employant un câble ou encore un système à engrenage.

Suivant le cas représenté, et de façon avantageuse, les moyens de préhension aptes à animer les moyens d'actionnement ont un déplacement sensiblement suivant la direction longitudinale 30 de la valve de sorte à optimiser l'ergonomie de l'ensemble. Un système de transformation de mouvement est ainsi prévu entre le moyen de préhension 14 et l'extrémité 3 mobile en rotation. Dans le cas illustré, le système de transformation de mouvement comporte la poulie 12 et le câble actionnant l'arbre tournant 9. La transformation de mouvement peut être produite par d'autres moyens d'actionnement.

Suivant le mode de réalisation plus précisément présentée aux figures 11 à 15, un deuxième câble 32 est formé de sorte à transmettre un mouvement opposé à celui produit par le câble de transmission. Ainsi, le mouvement de retour de la valve est as suré par une action du praticien dans le sens opposé au premier mouvement. Dans ce cas, le ressort de rappel 24, optionnellement présent, accompagne le mouvement induit par le câble complémentaire 32.

Le câble complémentaire 32 a l'avantage d'assurer un retour en position optimale sans nécessiter un surdimensionnement du ressort de rappel 24 et en contrant les éventuels effets de collage ou de déformation rémanente du matériau de la portion souple 1.

De la figure 11, on comprend que le chemin du câble complémentaire 32, partiellement illustré, débute au niveau d'un siège 40a ou 40b d'accrochage de l'extrémité du câble sur le moyen de préhension 14 et passe par une poulie de retour 34 et une poulie de renvoi d'angle commune avec le câble de transmission ou coaxiale à celle-ci.

L'autre extrémité du câble complémentaire 32 est fixée et enroulée dans une gorge 33 formée additionnellement dans l'arbre tournant 9. Les câbles sont bien entendu enroulés dans les gorges 18 et 33 de sorte à ce que la rotation de l'arbre tournant 9 engendre simultanément l'enroulement de l'un et le déroulement de l'autre.

La figure 15 montre une autre caractéristique avantageuse de l'invention qui peut être mise en oeuvre en complément ou séparément des caractéristiques précédentes. Dans ce cadre, des rangées de dents 35a, 35b sont formées de part et d'autre des bords de la glissière 15 dans laquelle coulisse le moyen de préhension 14. En formant un doigt 36 monté élastiquement, par une zone déformable élastiquement 37, sur le moyen de préhension 14, on peut fixer en position précisément le moyen de préhension 14 et, par conséquent, le degré d'ouverture de la valve. Les dents présentent une partie inclinée de glissement du doigt 36 et une partie verticale définissant une zone de blocage du doigt 36.

Ce système denté, ou un autre système de blocage du moyen de préhension 14, permet à l'utilisateur d'appliquer un effort élevé sur le câble de transmission (ou le câble complémentaire 32) et de maintenir cet effort pour obtenir une torsion forcée de la portion souple 1. De la sorte, une étanchéité optimale peut être produite, même dans le cas où l'objet à introduire est de diamètre variable : la torsion étant forcée, la portion souple 1 s'adapte à une éventuelle réduction du diamètre de l'objet.

Suivant une variante non illustrée, les bords de la glissière 15 sont gradués ou portent des indications chiffrées sur le degré de rotation de l'extrémité mobile 3 ou sur le niveau d'étanchéité. Sur ce dernier point, il faut noter que les instruments chirurgicaux à introduire sont généralement d'un diamètre standard (3 ou 4 diamètres différents en général) de sorte que l'on peut déduire le niveau d'étanchéité (par exemple exprimé en frenchs) de la position du moyen de préhension 14 dans la glissière, en connaissant le diamètre de l'objet à introduire.

Les indications sur la glissière peuvent cependant aussi être limitées au repérage du diamètre de rotation de l'extrémité mobile 3. Par exemple, il est utile de repérer au moins une rotation de 360°.

On notera que le sens d'actionnement du moyen de préhension n'est pas limitatif : suivant un premier cas, on pousse sur le moyen 14 vers l'extrémité distale de l'introduction pour ouvrir la valve. Dans un second cas, on pousse pour fermer la valve.

Enfin, un raidisseur 39 situé au niveau de l'extrémité proximale 31 de l'instrument permet de recevoir les objets à introduire au travers de la valve en assurant leur guidage parfaitement centré sur l'axe de la valve.

### REFERENCES

1. Portion souple
2. Extrémité
3. Extrémité
4. Corps
5. Tube
6. Ouverture latérale
7. Cavité
8. Bague fixe
9. Arbre tournant
10. a, 10b. Bague interne
11. Capot
12. Poulie
13. Axe
14. Moyen de préhension
15. Glissière
16. a, 16b. Rainure
17. Découpe
18. Gorge
19. Zone de fixation
20. Trou
21. Face avant
22. Face arrière
23. Guide
24. Ressort de torsion
25. Joint d'étanchéité
26. Joint d'étanchéité
27. Goupille
28. Molette
29. Valve additionnelle
30. Axe longitudinal
31. Extrémité proximale
32. Câble complémentaire
33. Gorge additionnelle
34. Poulie de retour
35. a, 35b. Rangées de dents
36. Doigt
37. Zone élastique
38. Patte
39. Raidisseur
40. a, 40b. Siège d'extrémité de câbles

## Revendications

1. Valve pour instrument chirurgical ou médical comportant :
- un passage au moins partiellement obturable par déformation en torsion d'une portion souple (1) de la paroi du passage,
- des moyens d'actionnement en rotation d'une extrémité mobile (3) de la portion souple (1) aptes à engendrer la torsion, dans laquelle :
- les moyens d'actionnement comportent un câble de transmission de mouvement à l'extrémité mobile (3) de la portion souple (1),
- un bout du câble de transmission est fixé à un moyen de préhension (14) déplaçable par un opérateur,
la valve comportant un organe de renvoi d'angle pour dévier le câble de transmission,
**caractérisée par le fait que** l'organe de renvoi d'angle et le moyen de préhension (14) sont configurés pour que le moyen de préhension (14) soit déplaçable en translation selon l'axe longitudinal de la valve.

2. Valve selon la revendication 1, **caractérisée par le fait**
**que** l'organe de renvoi d'angle est une poulie (12).

3. Valve selon l'une quelconque des revendications précédentes **caractérisée par le fait qu'**elle comporte des moyens de rappel en position par défaut de l'extrémité mobile (3)

4. Valve selon la revendication 3, **caractérisée par le fait**
**que** les moyens de rappel sont un ressort de torsion (24) dont une extrémité est fixe et l'autre extrémité est solidaire en rotation de l'extrémité mobile (3) de la portion souple (1).

5. Valve selon la revendication 3 ou 4, **caractérisée par le fait**
**que** les moyens de rappel sont configurés pour obturer le passage de la valve par défaut.

6. Valve selon la revendication 3 ou la revendication 4 **caractérisée par le fait**
**qu'**elle comporte des moyens de blocage de la valve en position ouverte.

7. Valve selon la revendication 6 **caractérisée par le fait**
**que** les moyens de blocage comportent une goupille (27) d'arrêt des moyens d'actionnement

8. Valve selon l'une quelconque des revendications 1 à 7, **caractérisée par le fait**
**que** l'extrémité mobile (3) est solidaire en rotation d'un arbre tournant (9) auquel est fixé un bout du câble de transmission.

9. Valve selon la revendication 8, **caractérisée par le fait**
**que** l'arbre tournant (9) comporte une gorge (18) de fixation et d'enroulement du câble de transmission.

10. Valve selon l'une quelconque des revendications 1 à 9 comportant un câble complémentaire (32) apte à transmettre, lorsqu'il est sollicité en traction, un mouvement à l'extrémité mobile (3) de la portion souple (1) dans un sens inverse à celui du câble de transmission.

11. Valve selon la revendication 10 dans laquelle le câble complémentaire (32) est guidé par une poulie de retour (34) et une poulie de renvoi d'angle.

12. Valve selon la revendication 10 ou la revendication 11 dans laquelle l'extrémité mobile (3) est solidaire en rotation d'un arbre tournant (9) comportant une gorge (18) de fixation et d'enroulement du câble de transmission et une gorge additionnelle (33) de fixation et d'enroulement du câble complémentaire (32).

13. Valve selon la revendication 1 dans laquelle le moyen de préhension (14) est déplaçable en translation dans une glissière (15) dont les bords comportent au moins une rangée de dents (35a, 35b) de rétention d'un doigt monté élastiquement sur le moyen de préhension.

14. Instrument médical ou chirurgical **caractérisé par le fait qu'**il comporte une valve selon l'une quelconque des revendications 1 à 13.

## Patentansprüche

1. Ventil für ein chirurgisches oder medizinisches Instrument, welches folgendes umfasst:
- einen Durchgang, der zumindest teilweise durch Torsionsverformung eines Ventilabschnitts (1) der Durchgangswand verschließbar ist,
- Betätigungsmittel zur Rotation eines beweglichen Endes (3) des nachgiebigen Abschnitts (1), die in der Lage sind, die Torsion zu erzeugen, in dem:
- die Betätigungsmittel ein Kabel zur Übertragung der Bewegung an das bewegliche Ende (3) des nachgiebigen Abschnitts (1) umfassen,
- ein Ende des Übertragungskabels an einem Greifmittel (14) befestigt ist, das von einem Bediener bewegt werden kann,
wobei das Ventil ein Eckumlenkelement zum Umlenken des Übertragungskabels umfasst,
**dadurch gekennzeichnet, dass** das Eckumlenkelement und das Greifmittel (14) so gestaltet sind, dass das Greifmittel (14) auf der Längachse des Ventils translatorisch verschiebbar ist.

2. Ventil nach Anspruch 1, **dadurch gekennzeichnet, dass** das Eckumlenkelement eine Rolle (12) ist.

3. Ventil nach einem der vorhergehenden Ansprüche, **dadurch gekennzeichnet, dass** es Mittel zum Rückstellen des beweglichen Endes (3) in die Standardposition aufweist.

4. Ventil nach Anspruch 3, **dadurch gekennzeichnet, dass** die Rückstellmittel aus einer Torsionsfeder (24) bestehen, deren eines Ende feststehend ist und deren anderes Ende mit dem beweglichen Ende (3) des nachgiebigen Abschnitts (1) drehfest verbunden ist.

5. Ventil nach Anspruch 3 oder 4, **dadurch gekennzeichnet, dass** die Rückstellmittel so gestaltet sind, dass der Durchgang des Ventils grundsätzlich verschlossen wird.

6. Ventil nach Anspruch 3 oder Anspruch 4, **dadurch gekennzeichnet, dass** es Mittel zum Feststellen des Ventils in offener Position umfasst.

7. Ventil nach Anspruch 6, **dadurch gekennzeichnet, dass** die Feststellmittel einen Arretierstift (27) für die Betätigungsmittel umfassen.

8. Ventil nach einem der Ansprüche 1 bis 7, **dadurch gekennzeichnet, dass** das bewegliche Ende (3) drehfest mit einer rotierenden Welle (9) verbunden ist, an welcher ein Stück des Übertragungskabels befestigt ist.

9. Ventil nach Anspruch 8, **dadurch gekennzeichnet, dass** die rotierende Welle (9) eine Nut (18) zur Befestigung und zum Aufwickeln des Übertragungskabels aufweist.

10. Ventil nach einem der Ansprüche 1 bis 9, welches ein zusätzliches Kabel (32) aufweist, das im Falle einer Zugbeanspruchung eine Bewegung in der dem Übertragungskabel entgegen gesetzten Richtung an das bewegliche Ende (3) des nachgiebigen Abschnitts (1) übertragen kann.

11. Ventil nach Anspruch 10, in dem das zusätzliche Kabel (32) über eine Umkehrrolle (34) und eine Eckumlenkrolle geführt wird.

12. Ventil nach Anspruch 10 oder Anspruch 11, in dem das bewegliche Ende (3) drehfest mit einer rotierenden Welle (9) mit einer Nut (18) zum Befestigen und Aufwickeln des Übertragungskabels und einer zusätzlichen Nut (33) zum Befestigen und Aufwickeln des zusätzlichen Kabels (32) verbunden ist.

13. Ventil nach Anspruch 1, in dem das Greifmittel (14) in einer Führungsbahn (15), deren Ränder mindestens eine Reihe von Zähnen (35a, 35b) zum Rückhalten eines auf dem Greifmittel federnd montierten Fingers umfassen, translatorisch bewegt werden kann.

14. Medizinisches oder chirurgisches Instrument, **dadurch gekennzeichnet, dass** es ein Ventil nach einem der Ansprüche 1 bis 13 umfasst.

## Claims

1. A valve for a surgical or medical instrument comprising:
- a passage which can be at least partially closed by the distortion of a flexible portion (1) of the passage wall,
- means for actuating in rotation a mobile end (3) of the flexible portion (1), so adapted as to generate torsion, wherein:
- the actuating means comprises a cable for transmitting movement to the mobile end (3) of the flexible portion (1),
- one end of the transmission cable is attached to gripping means (14) which can be moved by an operator,
with the valve comprising an angle transmission member for deflecting the transmission cable,
**characterized in that** the angle transmission member and the gripping means (14) are so configured that the gripping means (14) can be moved in translation along the longitudinal axis of the valve.

2. A valve according to claim 1, **characterized in that** the angle transmission member is a pulley (12).

3. A valve according to any one of the preceding claims, **characterized in that** it comprises means for returning the movable end (3) to the default position thereof.

4. A valve according to claim 3, **characterized in that** the return means is a torsion spring (24), one end of which is stationary and the other end rotates integrally with the mobile end (3) of the flexible portion (1).

5. A valve according to claims 3 or 4, **characterized in that** the return means is so configured as to close the default passage of the valve.

6. A valve according to claim 3 or claim 4, **characterized in that** it comprises means for locking the valve in the open position.

7. A valve according to claim 6, **characterized in that** the locking means comprises a pin (27) for stopping the actuating means.

8. A valve according to any one of claims 1 to 7, **characterized in that** the mobile end (3) rotates integrally with a rotating shaft (9) which one end of the transmission cable is connected to.

9. A valve according to claim 8, **characterized in that** the rotating shaft (9) has a groove (18) for fixing and winding the transmission cable.

10. A valve according to any one of claims 1 to 9, comprising an additional cable (32) so adapted as to transmit, when tension-loaded, a movement to the mobile end (3) of the flexible portion (1) in a direction opposite that of the transmission cable.

11. A valve according to claim 10, wherein the additional cable (32) is guided by a return pulley (34) and an angle transmission pulley.

12. A valve according to claim 10 or claim 11, wherein the mobile end (3) rotates integrally with a rotating shaft (9) having a groove (18) for fixing and winding the transmission cable and an additional groove (33) for fixing and winding the additional cable (32).

13. A valve according to claim 1, wherein the gripping means (14) can be moved in translation in a slide (15), the edges of which comprise at least one row of teeth (35a, 35b) for holding a finger elastically mounted on the gripping means.

14. A medical or surgical instrument **characterized in that** it comprises a valve according to any one of claims 1 to 13.
